# FASCICULE DE BREVET EUROPEEN

(11) **EP 0 787 137 B1**
(45) Date de publication et mention de la délivrance du brevet: **23.01.2002**
(21) Numéro de dépôt: 95934205.6
(22) Date de dépôt: 19.10.1995
(51) Int. Cl.: C07D 263/44

(54) **PROCEDE POUR LA PREPARATION DE N-CARBOXYANHYDRIDES D'ACIDES AMINES A PARTIR D'ACIDES AMINES N-(N'-NITROSOCARBAMOYLES)**
VERFAHREN ZUR HERSTELLUNG VON N-CARBOXYANHYDRIDAMINOSÄUREN AUS N-(N'-NITROSOCARBAMOYL)-AMINOSÄUREN
PROCESS FOR THE PREPARATION OF N-CARBOXYANHYDRIDE AMINO ACIDS FROM N-(N'-NITROSOCARBAMOYL)AMINO ACIDS

(30) Priorité: 24.10.1994 FR 9412779
(43) Date de publication de la demande: 06.08.1997
(60) Demande divisionnaire: 00109844.1
(73) Titulaire: Degussa AG, 40474 Düsseldorf (DE)
(72) Inventeur: COMMEYRAS, Auguste, F-34170 Clapiers (FR); COLLET, Hélène, F-34000 Montpellier (FR); MION, Louis, F-34000 Montpellier (FR); BENEFICE, Sylvie, F-34080 Montpellier (FR); CALAS, Patrick, F-34000 Montpellier (FR); CHOUKROUN, Henri, F-34830 Clapiers (FR); TAILLADES, Jacques, F-34170 Clapiers (FR); BIED, Catherine, F-34090 Montpellier (FR)
(86) Numéro de dépôt international: FR9501380
(87) Numéro de publication internationale: WO9612729

(56) Documents cités:
- CHEMICAL ABSTRACTS, vol. 92, no. 15, 14 Avril 1980 Columbus, Ohio, US; abstract no. 129280v, V V LUTSENKO: "Acylation of alkyl esters of alpha-amino acids by nitrosohydantoic acid" page 741; XP002007190 & ZH. ORG. KHIM., vol. 15, no. 9, 1979, pages 1973-1976,
- CHEMICAL ABSTRACTS, vol. 75, no. 17, 25 Octobre 1971 Columbus, Ohio, US; abstract no. 110012m, V V LUTSENKO ET AL.: "N-Nitrosoureides. II. Nitrosation of some alpha-ureido acids" page 410; XP002007191 & ZH. ORG. KHIM., vol. 7, no. 6, 1971, pages 1152-1159,
- JOURNAL OF THE AMERICAN CHEMICAL SOCIETY, vol. 93, no. 11, 2 Juillet 1971, DC US, pages 2746-2754, XP002007186 R HIRSCHMANN ET AL.: "The controlled synthesis of peptides in aqueous medium. The preparation and use of novel alpha-amino acid N-Carboxyanhydride"
- JOURNAL OF THE CHEMICAL SOCIETY, PERKIN TRANSACTIONS 1, no. 17, 1977, LETCHWORTH GB, pages 1911-1915, XP002007187 K INOUYE & K WATANABE: "Formation and degradation of urea derivatives in the azide method of peptide synthesis. Part 2. Acidolytic degradation of urea derivatives"
- JOURNAL OF THE CHEMICAL SOCIETY, PERKIN TRANSACTIONS 1, no. 17, 1977, LETCHWORTH GB, pages 1905-1911, XP002007188 K INOUYE ET AL.: "Formationa nd degradation of urea derivatives in the azide method of peptide synthesis. Part 1. The Curtius rearrangement and urea formation"
- TETRAHEDRON LETTERS, vol. 435, no. 42, 17 Octobre 1994, OXFORD GB, pages 7731-7734, XP002007189 S PAIK & E H WHITE: "N-Acyl-N-Nitrosoamino acids and peptides "
- CHEMICAL ABSTRACTS, vol. 101, no. 15, 8 Octobre 1984 Columbus, Ohio, US; abstract no. 125184y, M SAKURAI & H YANAGAWA: "Prebiotic synthesis of amino acids from formaldehyde and hydroxylamine in a modified sea medium" page 258; XP002007192 & ORIGINS LIFE, vol. 14, no. 1-4, 1984, pages 171-176,
- CHEMICAL ABSTRACTS, vol. 84, no. 17, 26 Avril 1976 Columbus, Ohio, US; abstract no. 122296f, I K ROMANOVSKAYA ET AL.: "Solid-phase synthesis of (1-asparagine, 5-valine, 8-alanine) and (1-hydantoic acid, 5-valine, 8-alanine) angiotensin II" page 588; XP002007193 & BIOORG. KHIM., vol. 1, no. 9, 1975, pages 1257-1262,

## Description

L'invention concerne un nouveau procédé de synthèse de N-carboxyanhydrideaminoacides pouvant constituer des produits intermédiaires dans le cadre de la synthèse peptidique.

Les peptides sont des molécules biologiquement actives d'un intérêt considérable et leur synthèse a été étudiée depuis fort longtemps, cette synthèse pouvant s'effectuer par des méthodologies diverses, aussi bien en phase homogène qu'en phase solide ou supportée. Dans un cas comme dans l'autre, ces méthodologies utilisent des acides aminés protégés sur une fonction, créent ou allongent le peptide sur la fonction libre restante au moyen d'un acide aminé bloqué, débloquent cette dernière fonction et poursuivent ainsi pas à pas la construction de la chaîne. Pour avoir un peptide libre les fonctions terminales doivent évidemment être débloquées. Le solvant utilisé est essentiellement un solvant organique. De telles méthodologies tout en étant efficaces sont néanmoins très lourdes.

L'invention a pour objectif la mise au point d'un nouveau procédé de synthèse de N-carboxyanhydrideaminoacides.

A cet effet, le procédé conforme à l'invention se caractérise en ce que
(a) on prépare un N-(N'-nitrosocarbamoyl)aminoacide de formule : 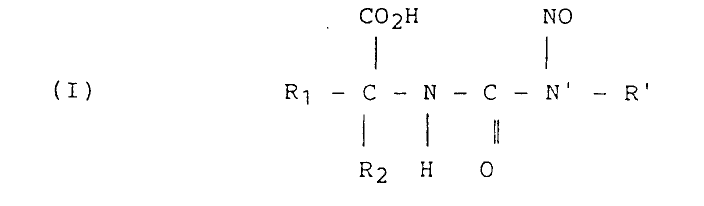 où R1 et R2 sont un hydrogène ou un radical alkyl, cycloalkyl, aryl ou aryl alkyl, substitué ou non par une ou des fonctions alcool, thiol, amine, sulfure, acide ou amide, et R' est un radical du groupe : H, CH3, CH2CH2Cl, et
(b) on décompose le composé (I) en N2, R'OH et N-carboxyanhydrideaminoacide de formule :

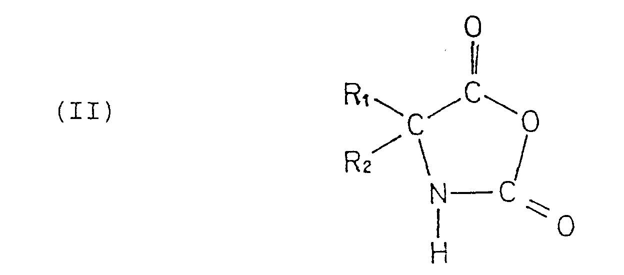

Selon un mode de mise en oeuvre préféré, on choisit comme composé de départ un composé (I) dans lequel R' est un hydrogène, en vue d'obtenir, au terme de la décomposition (b), de l'eau comme sous-produit de décomposition.

On peut choisir comme radical R1 un résidu d'acide aminé naturel et comme radical R2 l'hydrogène ; on peut également choisir comme radical R1 un résidu d'acide aminé modifié et comme radical R2 un groupement méthyl, éthyl ou propyl.

Le procédé de l'invention est avantageusement mis en oeuvre selon les deux modes opératoires décrits ci-après.

En premier lieu, la réaction de décomposition (b) peut être réalisée dans un solvant organique anhydre.

Un autre mode opératoire consiste à opérer la décomposition (b) en milieu aquo-organique à un pH sensiblement compris entre 6 et 10.

Les conditions de température de la décomposition (b) ne paraissent pas critiques en pratique, on peut opérer à température ambiante toutefois, dans certains cas, une température plus élevée peut être choisie pour la décomposition afin de la favoriser (50°C par exemple).

Il est à noter que la synthèse des N-(N'-nitrosocarbamoyl)aminoacides (I) est décrite dans la littérature (DE-OS 2,615,594, Japan Kokai 78 103,441, EPA 88 105 584 2 pour R' = H et Bul. Chem. Soc. Japan, vol. 48 (4), 1333-1334 (1975), Arch. Pharm. Weinheim 314,910-917 (1981), 317,481-487 (1984), 322,863-872 (1989), pour R' = CH₃, ClCH₂CH₂-... ). Ils sont décrits dans le cas où R' = H comme se décomposant en milieu acide à des pH inférieurs à 4 en donnant l'acide aminé correspondant, de l'azote et de l'anhydride carbonique (voir EPA 88 105 584.2). Ils sont jusqu'à présent utilisés pour hydrolyser la fonction carbamoyle. Les dérivés substitués (R' = CH₃, ClCH₂CH₂-...) font quant à eux partie de la famille des nitrosourées N'-alkylées. Leurs propriétés antitumorales ont été étudiées, elles résulteraient de leur pouvoir alkylant (Arch Pharm 32∼, 863-872 (1989).

Selon une caractéristique du procédé de l'invention, les composés (I) peuvent être synthétisés directement dans le milieu réactionnel anhydre (dioxane, acétonitrile, ... ), aqueux à pH inférieur à 4 ou aquo-organique à pH inférieur à 4 par action d'un agent nitrosant tel que °NO, N0₂H, N₂0₃, N₂0₄, ... utilisé pur ou en mélange avec un agent oxydant (oxygène, ou tout autre agent tel que Fe3⁺, Cu2⁺, couples métalliques, ou oxydation électrochimique) sur un N-[N'-(R')carbamoyl]aminoacide de formule :

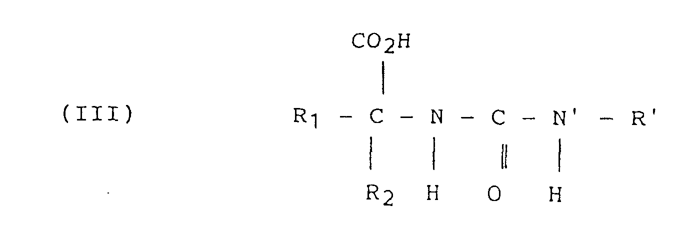

Un intermédiaire réactionnel dans cette réaction a été déterminé comme étant le composé N-nitroso-N-carbamoylaminoacide de formule (IV) qui, dans des conditions opératoires différentes de celles utilisées dans ce procédé, peut se décomposer en partie pour donner un α-hydroxy acide :

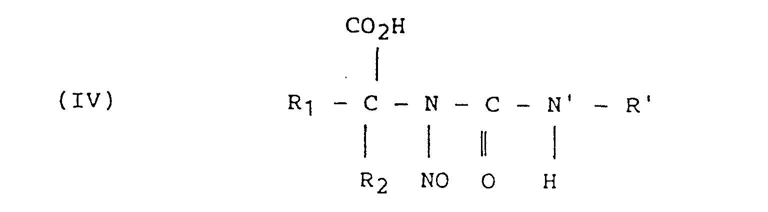

Les composés (I) peuvent également être préparés in situ dans le milieu réactionnel en deux étapes :
. en faisant d'abord réagir à un pH inférieur à 8 de l'acide isocyanique (HNCO), un sel de cet acide ou un isocyanate de méthyle ou de chloroéthyle sur un aminoacide pour obtenir un N-[N'-(R')carbamoyl]aminoacide de formule : 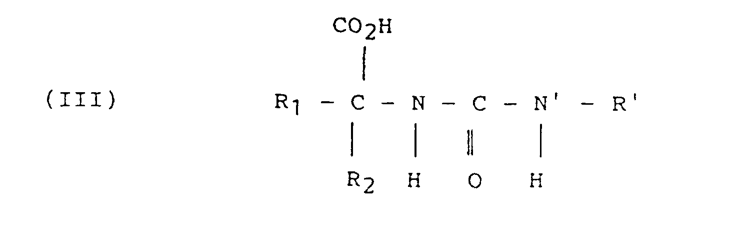
. et en faisant ensuite réagir un agent nitrosant sur le N-[N'-(R)carbamoyl]aminoacide formé.

Il faut souligner que les aminoacides utilisés peuvent être de chiralité D ou L, ou racémiques. Lorsque les composés utilisés sont énantiomériquement purs, ils ne se racémisent pas au cours de la réaction.

Lorsque la décomposition du N-(N'-nitrosocarbamoyl)aminoacide (I) est réalisée dans un solvant organique anhydre, on recueille un N-carboxyanhydrideaminoacide de plus grande stabilité, après dégagement d'azote et séparation du solvant organique contenant l'eau formée (ces conditions opératoires évitent une hydrolyse du composé N-carboxyanhydrideaminoacide).

Le procédé de l'invention est illustré ci-après au moyen d'exemples de mise en oeuvre.

### EXEMPLE 1 : Synthèse du N- (N'-nitrosocarbamoyl)valine (compose I dans lequel R1 = iPr, R₂ = H, R' = H) à partir de N-carbamoylvaline (compose III avec les radicaux précités)

1 mmole (160 mg) de N-carbamoylvaline racémique est placée sous agitation dans 20 ml de dioxane anhydre à 20°C. La solution est balayée pendant 1/2 heure par un courant d'azote. A cette solution est alors ajouté un mélange gazeux de 66 ml de NO et 26 ml d'air (70 % de NO). Après 1/4 d'heure de réaction, le solvant et l'excédent d'agent nitrosant sont chassés sous pression réduite conduisant à une masse incolore (190 mg) qui cristallise. La solution est analysée en HPLC (chromatographie liquide haute pression), sur une colonne C18 avec comme éluant une solution eau/acétonitrile 9/1 contenant 0,05 % CF3COOH et détectée en UV à 220 nm. L'analyse isocratique à 1 ml/mn donne deux signaux a 22 et 28 mn d'intensité relative 30/70. Une nouvelle analyse du même milieu réactionnel 60 minutes après la précédente montre que l'intensité relative de ces deux signaux s'inverse 70/30. L'analyse en RMN ¹H (résonance magnétique nucléaire du proton) de ces deux situations a permis d'établir que les produits correspondant à ces deux signaux sont respectivement, la N-(N'-nitrosocarbamoyl)valine (I) RMN ¹H (DMSO-d6 + TMS) ; δ (ppm) : 0,85 (d, 3H, CH3) ; 0,95 (d, 3H, CH₃) ; 2,1 (m, 1H, CH) ; 4,3 (dd, 1H, CH) ; 7,3 (s, 1H, NH) ; 9,15 (s, 1H, NH) et la N-nitroso-N-carbamoylvaline (IV) RMN ¹H (DMSO-d6 + TMS) ; δ (ppm) = 0,85 (d, 3H, CH₃) ; 0,95 (d, 3H, CH₃) ; 2,1 (m, 1H, CH) ; 4, 3 (dd, 1H, CH) ; 9,0 (s, 2H, NH₂).

Une troisième analyse 60 minutes après la précédente montre la disparition du signal à 28 mn au profit du signal à 22 mn correspondant ainsi à la formation quasi quantitative de la N-(N'-nitrosocarbamoyl)valine (I), même si dans ce dernier chromatogramme commence à apparaître avec une très faible intensité un signal à 17 mn que l'on analyse dans l'exemple 2 ci-dessous.

### EXEMPLE 2 : Synthèse du N-carboxyanhydridevaline (II ; R1 = iPr, R2 = H) à partir de N-(N'-nitrosocarbamoyl)valine (I)

1 mmole de N-(N'nitrosocarbamoyl)valine (I) provenant de l'exemple précédent placée dans 20 ml de dioxane anhydre sous une atmosphère d'hélium est maintenue à 30°C pendant 30 minutes au cours desquelles on observe un dégagement gazeux au sein de la solution. Ce dégagement est analysé en spectroscopie de masse comme étant de l'azote moléculaire (Masse 28). L'analyse de la solution en HPLC montre alors la présence d'un seul signal à 17 mn et la disparition du signal à 22 mn. Le milieu réactionnel est ensuite évaporé sous pression réduite de façon à éliminer le solvant et l'eau formée au cours de la réaction. Le produit brut (145 mg) cristallisé est identifié, en le comparant à un échantillon authentique, comme étant le N-carboxyanhydridevaline.

TF = 75°C produit brut, 81°C après recristallisation. RMN ¹H (DMSO-d6 + TMS) ; δ (ppm) : 0,25 (d, 3H, CH3) ; 0,95 (d, 3H, CH3) ; 2,1 (m, 1H, CH) ; 9,15 (s, 1 H, NH).

### EXEMPLE 3 : Synthèse du N-carboxyanhydridevaline (II ; R1 = iPr, R2 = H) à partir de N-carbamoylvaline (III) dans un solvant organique

1 mmole de N-carbamoylvaline est placée dans 20 ml d'acétonitrile (contenant moins de 0,1 % d'eau) à température ambiante et sous atmosphère d'azote. Le mélange nitrosant (66 ml NO + 26 ml d'air) est ajouté. Après 30 minutes de réaction, l'excès d'agent nitrosant et le solvant sont chassés sous pression réduite. L'analyse de la solution en HPLC du produit brut cristallisé, (PF 74°C) montre la présence d'un seul signal à 17 mn caractéristique du N-carboxyanhydridevaline. Son spectre de RMN est conforme à celui donné dans l'exemple 2.

### EXEMPLE 4 : Synthèse du N-carboxyanhydridevaline (II ; R1 = iPr, R2 = H) à partir de N-carbamoylvaline (III) en milieu aquo-organique

1 mmole de N-carbamoylvaline est placée dans 20 ml d'eau, à température ambiante sous atmosphère d'azote. Le mélange nitrosant (264 ml NO + 104 ml d'air) est ajouté. Après 30 minutes de réaction, l'excès d'agent nitrosant et le solvant sont chassés sous pression réduite. L'analyse de la solution en HPLC du produit brut, montre la présence du signal à 17 mn caractéristique du Ncarboxyanhydridevaline ainsi que du signal à 4,8 mn de la valine dans le rapport 80/20.

### EXEMPLE 5 : Synthèse du N-carboxyanhydridevaline (II ; R1 = iPr, R2 = H) à partir de la valine (V) en milieu aquo-organique

1 mmole de valine et 3 mmole de cyanate de potassium sont ajoutés à 20 ml d'eau, à température ambiante (pH égal à 4). La formation de N-carbamoylvaline -(III) est suivie en HPLC. Lorsque la formation de Ncarbamoylvaline (III) est complète (au bout de 2 heures), le milieu est placé sous atmosphère d'azote et le mélange nitrosant (264 ml NO + 104 ml d'air) est ajouté. Après 30 minutes de réaction, l'excès d'agent nitrosant et le solvant sont chassés sous pression réduite. L'analyse HPLC, montre comme dans l'exemple 4 la présence d'un signal caractéristique du N-carboxyanhydridevaline ainsi que d'un signal d'intensité plus faible de la valine dans le rapport 80/20.

### EXEMPLE 6 : Synthèse de la N-(N'nitrosocarbamoyl)alanine (I ; R₁ = CH₃, R₂ = H, R' = H) puis sa décomposition en N-carboxyanhydridealanine (II)

1 mmole (132 mg) de D,L-N-carbamoylalanine (III) est mise en réaction comme dans l'exemple 1. L'analyse HPLC (éluant eau/acétonitrile 95/5 + 0,05 % CF₃CO₂H) montre comme pour la valine la formation d'une espèce sous contrôle cinétique à 15 mn évoluant ensuite vers une seconde espèce caractérisée par un signal 11 mn. Le spectre de RMN ¹H (DMSO-d6 + TMS) ; δ (ppm) 1,2 (d, 3H, CH3) ; 4,05 (q, H, CH) ; 8 (s, 1H, NH) ; 8,95 (s, 1H, NH) caractérise la N-(N'-nitrosocarbamoyl)alanine. Cette dernière espèce évolue à son tour pour donner quantitativement le D,L-N-carboxyanhydridealanine caractérisé par son temps de rétention HPLC à 6,9 mn, son point de fusion (60°C) et son spectre de RMN.

### EXEMPLE 7 : Synthèse du D,L-N-(N'-nitrosocarbamoyl)méthionine (I ; R₁ = CH₃S(CH₂)₂-, R₂ = H, R' = H) puis sa décomposition en N-carboxyanhydride méthionine (II)

1 mmole de N-carbamoylméthionine (III) (192 mg) est placée dans 20 ml d'acétonitrile et traitée selon le mode opératoire de l'exemple 1. Après 5 minutes de réaction, l'excès d'agent nitrosant et le solvant sont éliminés sous pression réduite.

L'analyse de la solution en HPLC montre :
- un pic de très faible intensité à 28 mn caractérisant la présence résiduelle de la N-nitroso-N-carbamoylméthionine (IV),
- un pic très intense à 22 mn caractérisant la N- (N'-nitrosocarbamoyl) méthionine (I) RMN ¹H (DMSO- 6 + TMS) ; δ (ppm) : 2,02 (m, 2H, CH2) 2,06 (s, 3H, CH3) ; 2,55 (t, 2H, CH2) ; 4,58 (t, 1H, CH) ; 7,9 (s, 1H, NH) ; 8,2 (s, 1H, NH),
- un pic également très intense à 17 mn caractérisant le N-carboxyanhydrideméthionine (II) (RMN ¹H (DMSO-d6 + TMS) ; δ (ppm) : 2,02 (m, 2H, CH2) ; 2,06 (s, 3H, CH3) ; 2, 55 (t, 2H, CH2) ; 4, 6 (t, 1 H, CH) ; 9, 1 (s, 1H, NH).

Dans le cas de la méthionine, la décomposition totale du composé (I) en composé (II) nécessite un apport énergétique supérieur aux exemples précédents (50°C plusieurs heures).

L'hydrolyse du composé (II) ainsi obtenu conduit 93 % de méthionine et 7 % de méthionine sulfoxyde, montrant l'existence de possibilités de contrôle du pouvoir oxydant du milieu nitrosant utilisé.

## Revendications

1. Procédé de synthèse de N-carboxyanhydrideaminoacides
**caractérisé en ce que**
(a) on prépare un N-[N'-nitroso-N'-(R')carbamoyl]aminoacide de formule : 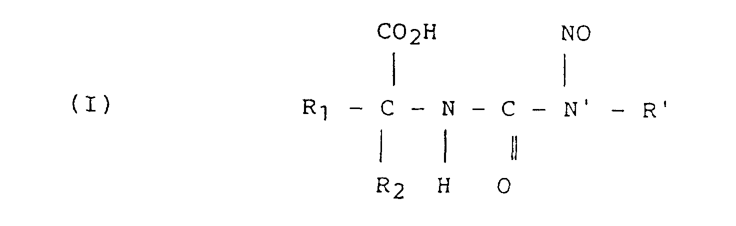 où R₁ et R₂ sont un hydrogène ou un radical alkyl, cycloalkyl, aryl ou aryl alkyl, substitué ou non par une ou des fonctions alcool, thiol, amine, sulfure, -acide ou amide,
et R' est un radical du groupe : H, CH₃, CH₂CH₂Cl, et
(b) on décompose le composé (I) en N₂, R'OH et N-carboxyanhydrideaminoacide de formule :

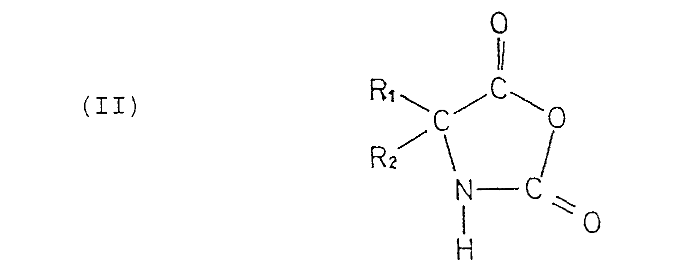

2. Procédé selon la revendication 1,
**caractérisé en ce que**
(a) on prépare un composé (I) dans lequel R' est un hydrogène, en vue d'obtenir, au terme de la décomposition
(b) (b), de l'eau comme sous-produit de décomposition.

3. Procédé selon la revendication 2,
**caractérisé en ce que**
(a) on prépare un composé (I) dans lequel R₁ est un résidu d'acide aminé et R₂ un hydrogène ou un groupement méthyl, éthyl ou propyl.

4. Procédé selon l'une des revendications 1, 2 ou 3,
**caractérisé en ce que**
(b) la décomposition du composé (I) est réalisée dans un solvant organique anhydre.

5. Procédé selon l'une des revendications 1, 2 ou 3,
**caractérisé en ce que**
(c) la décomposition du composé (I) est réalisée en milieu aquo-organique de pH sensiblement compris entre 6 et 10.

6. Procédé selon la revendication 5,
**caractérisé en ce qu'**
on utilise pour opérer la décomposition (b) un milieu aquo-organique tamponné au moyen de bicarbonate alcalin à pH compris entre 6,5 et 7,5.

7. Procédé selon l'une des revendications 1 à 6,
dans lequel
(a) le N-[N'-nitroso-N'-(R')carbamoyl]aminoacide est synthétisé directement dans le milieu réactionnel par action d'un agent nitrosant sur un N-[N'-(R')carbamoyl]aminoacide de formule :

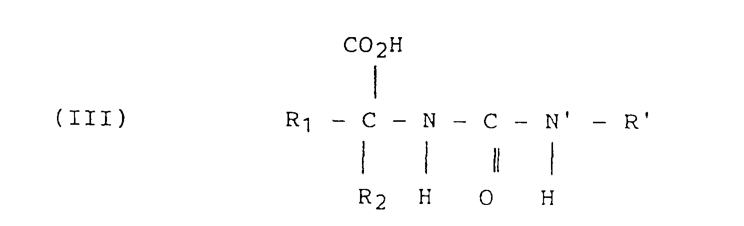

8. Procédé selon l'une des revendications 1 à 6,
dans lequel
(a) le N-[N'-nitroso-N'(R')carbamoyllaminoacide est synthétisé directement dans le milieu réactionnel en deux étapes :
. en faisant d'abord réagir de l'acide isocyanique, un sel de cet acide ou un isocyanate de méthyle ou de chloroéthyle sur un aminoacide pour obtenir un N-[N'-(R')carbamoyllaminoacide de formule : 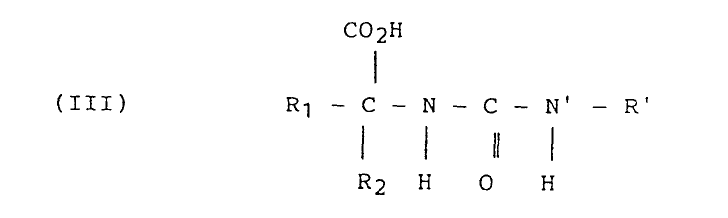
. et en faisant ensuite réagir un agent nitrosant sur le N-[N'-(R')carbamoyllaminoacide formé.

9. Procédé de synthèse du composé N-carboxyanhydrideaminoacide de formule : 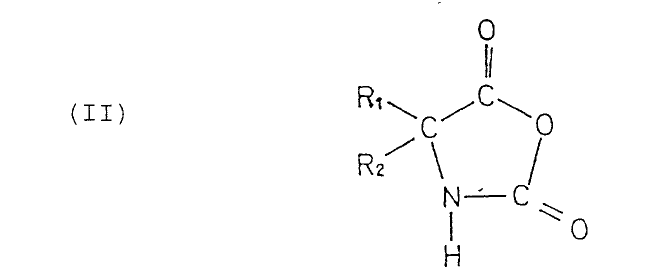 **caractérisé en ce qu'**
on décompose un N-(N-nitrosocarbamoyl)aminoacide de formule : 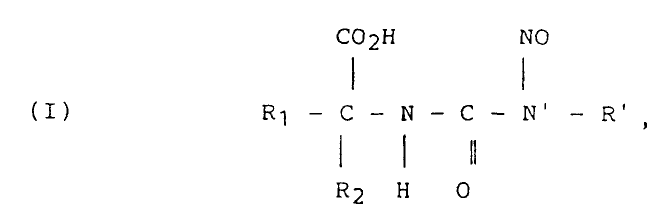 dans lequels R₁, R₂ et R' sont tels/que définis dans la revendication 1, et on recueille le N-carboxyanhydride après dégagement d'azote.

10. Procédé selon la revendication 9,
**caractérisé en ce que**
la décomposition du composé (I) est réalisée dans un solvant organique anhydre, ce solvant contenant l'eau formée étant séparé en fin d'opération.

## Claims

1. Method for synthesizing N-carboxyanhydride amino acids **characterized in that**
(a) an N-[N'-nitroso-N'-(R')carbamoyl] amino acid is prepared of formula: 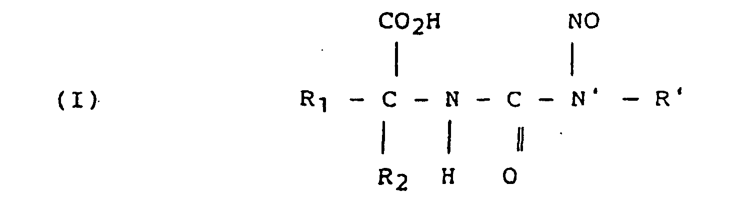 where R₁ and R₂ are a hydrogen or an alkyl, cycloalkyl, aryl or aryl alkyl radical, substituted or not by one or more alcohol, thiol, amine, sulfide, acid or amide functional groups,
and R' is a radical from the group: H, CH₃, CH₂CH₂Cl, and
(b) the compound (I) is decomposed into N₂, R'OH and N-carboxyanhydride amino acid of formula:

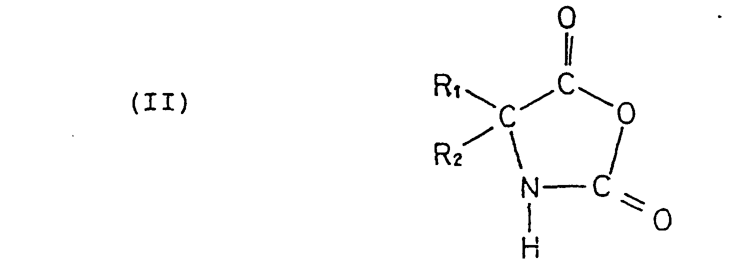

2. Method according to claim 1, **characterized in that**
(a) a compound (I) is prepared in which R' is a hydrogen so as to obtain water as a decomposition by-product at the end of the decomposition (b).

3. Method according to claim 2, **characterized in that**
(a) a compound (I) is prepared in which R₁ is an amino acid residue and R₂ is a hydrogen or a methyl, ethyl or propyl group.

4. Method according to one of claims 1, 2 or 3 **characterized in that**
(b) decomposition of the compound (I) is carried out in an anhydrous organic solvent.

5. Method according to one of claims 1, 2 or 3 **characterized in that**
(c) decomposition of the compound (I) is carried out in an aquo-organic medium with a pH substantially between 6 and 10.

6. Method according to claim 5, **characterized in that** in order to carry out the decomposition (b) an aquo-organic medium is used buffered by means of an alkaline bicarbonate to a pH between 6.5 and 7.5.

7. Method according to one of claims 1 to 6, wherein
(a) the N-[N'-nitroso-N'-(R')carbamoyl] amino acid is synthesized directly in the reaction medium by the action of a nitrosing agent on an N-[N'-(R')carbamoyl] amino acid of formula:

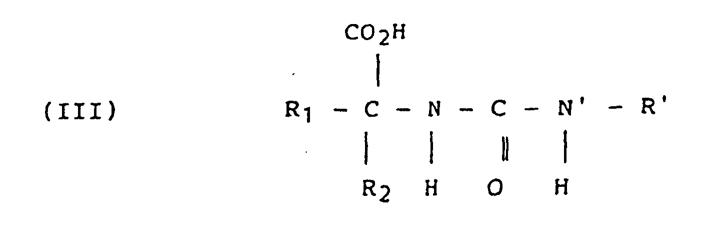

8. Method according to one of claims 1 to 6, wherein
(a) the N-[N'-nitroso-N' - (R') carbamoyl] amino acid is synthesized directly in the reaction medium in two steps:
. by first of all reacting isocyanic acid, a salt of this acid or a methyl or chloroethyl isocyanate with an amino acid so as to obtain an N-[N'-(R')carbamoyl] amino acid of formula: 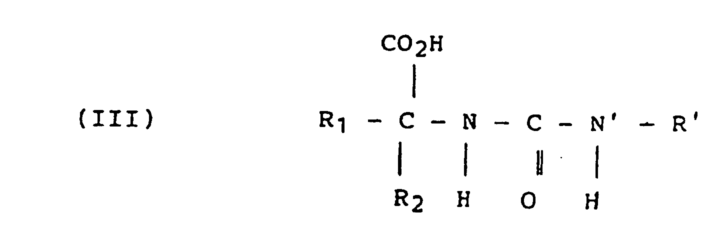
. and then by reacting a nitrosing agent with the N-[N'-(R')carbamoyl] amino acid formed.

9. Method for synthesizing the N-carboxyanhydride amino acid compound of formula: 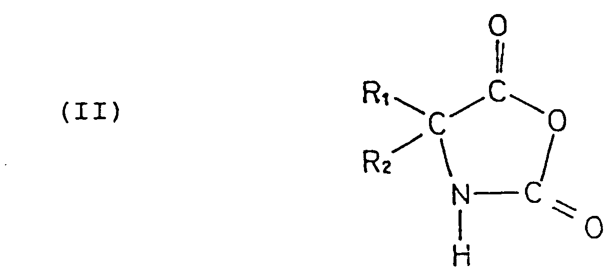 **characterized in that** an N-(N-nitrosocarbamoyl) amino acid of formula: 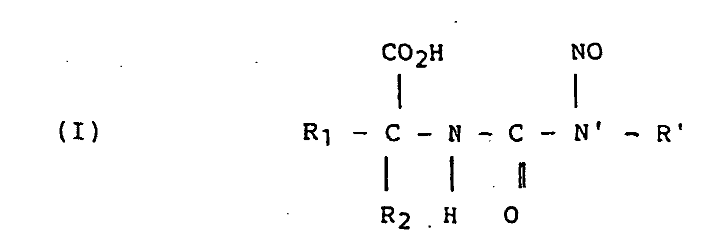 wherein R₁, R₂ and R' have the meaning like in claim 1 is decomposed and the N-carboxyanhydride is collected after nitrogen is evolved.

10. Method according to claim 9, **characterized in that** decomposition of the compound (I) is carried out in an anhydrous organic solvent, this solvent containing the water formed being separated at the end of the operation.

## Patentansprüche

1. Verfahren zur Synthese von N-Carboxyanhydridaminosäuren, **dadurch gekennzeichnet, dass**
(a) eine N-[N'-Nitroso-N'-(R')-carbamoyl]-aminosäure folgender Formel hergestellt wird: 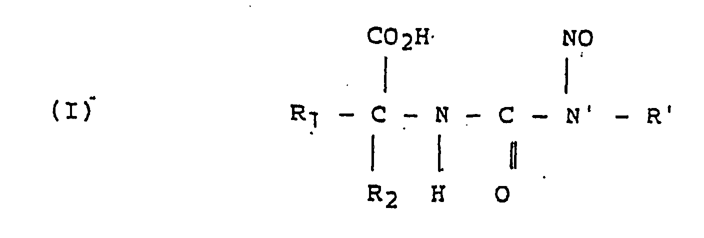 wobei R₁ und R₂ ein Wasserstoff oder ein Alkyl-, Cycloalkyl-, Aryl- oder Arylalkylradikal sind, substituiert oder nicht substituiert durch eine oder mehrere Alkohol-, Thiol-, Amin-, Sulfid-, Säure- oder Amidgruppen, und wobei R' ein Radikal der Gruppe: H, CH₃, CH₂CH₂Cl ist, und
(b) die Verbindung (I) in N₂, R'OH und eine N-Carboxyanhydridaminosäure folgender Formel gespalten wird:

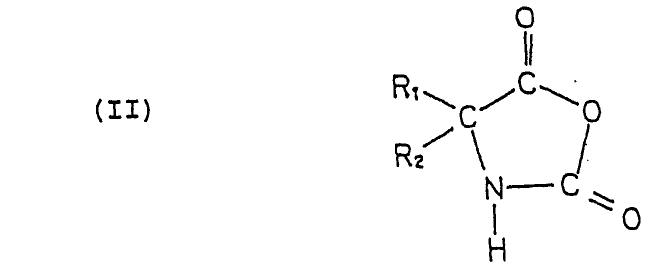

2. Verfahren nach Anspruch 1, **dadurch gekennzeichnet, dass**
(a) eine Verbindung (I) hergestellt wird, bei der R' ein Wasserstoff ist, wobei am Ende der Spaltung
(b) Wasser als Spaltungsnebenprodukt erhalten wird.

3. Verfahren nach Anspruch 2, **dadurch gekennzeichnet, dass**
(a) eine Verbindung (I) hergestellt wird, bei der R₁ ein Aminosäurerest und R₂ ein Wasserstoff oder eine Methyl-, Ethyl- oder Propylgruppe ist.

4. Verfahren nach einem der Ansprüche 1, 2 oder 3, **dadurch gekennzeichnet, dass**
(b) die Spaltung der Verbindung (I) in einem wasserfreien, organischen Lösungsmittel durchgeführt wird.

5. Verfahren nach einem der Ansprüche 1, 2 oder 3, **dadurch gekennzeichnet, dass**
(c) die Spaltung der Verbindung (I) in einem wässrig-organischen Medium mit einem im wesentlichen zwischen 6 und 10. gelegenen pH-Wert durchgeführt wird.

6. Verfahren nach Anspruch 5, **dadurch gekennzeichnet, dass** zur Durchführung der Spaltung (b) ein mittels alkalischem Hydrogencarbonat bei einem pH-Wert zwischen 6,5 und 7,5 gepuffertes wässrig-organisches Medium verwendet wird.

7. Verfahren nach einem der Ansprüche 1 bis 6, wobei
(a) die N-[N'-Nitroso-N'-(R')-carbamoyl]-aminosäure direkt in dem Reaktionsmedium durch Wirkung eines Nitrosierungsmittels auf eine N-[N'-(R')-carbamoyl]-aminosäure folgender Formel synthetisiert wird:

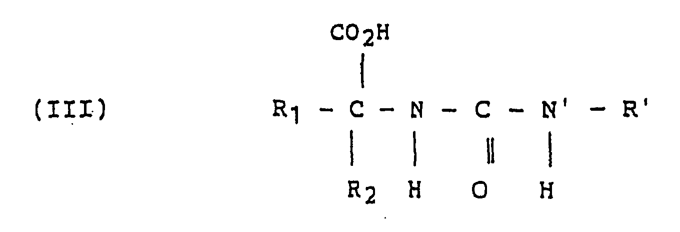

8. Verfahren nach einem der Ansprüche 1 bis 6, wobei
(a) die N-[N'-Nitroso-N'(R')-carbamoyl]-aminosäure direkt in dem Reaktionsmedium in zwei Schritten synthetisiert wird:
• indem man zunächst Isocyansäure, ein Salz dieser Säure oder ein Methyl- oder Chlorethylisocyanat mit einer Aminosäure reagieren lässt, wobei eine N-[N'-(R')-carbamoyl]-aminosäure folgender Formel erhalten wird: 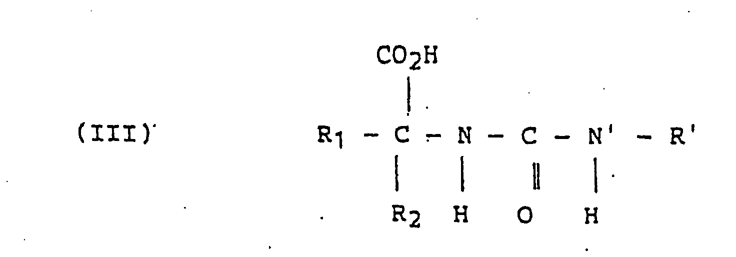
• und indem man anschließend ein Nitrosierungsmittel mit der gebildeten N-[N'-(R')-carbamoyl]-aminosäure reagieren lässt.

9. Verfahren zur Synthese der N-Carboxyanhydridaminosäure-Verbindung folgender Formel: 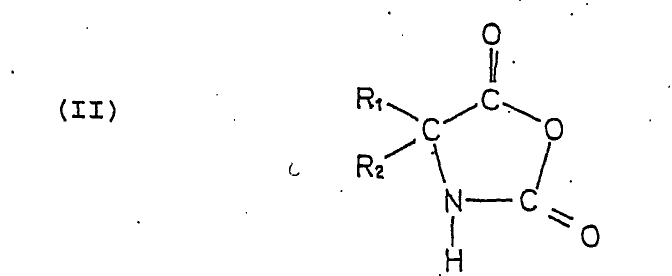 **dadurch gekennzeichnet, dass** eine N-(N-Nitrosocarbamoyl)-aminosäure folgender Formel gespalten wird: 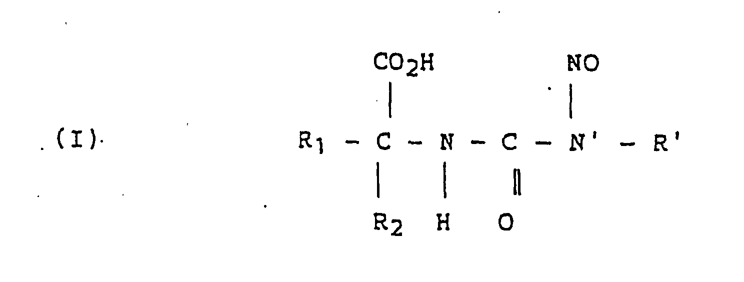 wobei die Reste R₁, R₂ und R' die Bedeutung wie in Anspruch 1 annehmen und nach Stickstoffabgabe das N-Carboxyanhydrid erhalten wird.

10. Verfahren nach Anspruch 9, **dadurch gekennzeichnet, dass** die Spaltung der Verbindung (I) in einem wasserfreien, organischen Lösungsmittel durchgeführt wird, wobei dieses Lösungsmittel, welches das gebildete Wasser enthält, am Ende des Verfahrens abgetrennt wird.
